(19) Europäisches Patentamt
European Patent Office
Office européen des brevets

(11) Veröffentlichungsnummer: **0 126 876**
**B1**

(12) **EUROPÄISCHE PATENTSCHRIFT**

(45) Veröffentlichungstag der Patentschrift: **04.10.89**

(21) Anmeldenummer: **84103033.1**

(22) Anmeldetag: **20.03.84**

(51) Int. Cl.⁴: **C 12 P 33/02,** C 07 J 1/00, C 07 J 5/00, C 07 J 7/00, C 07 J 71/00

# (54) Verfahren zur Herstellung von 3-Oxo-delta-1,4-steroiden.

(30) Priorität: **29.03.83 DE 3311985**

(43) Veröffentlichungstag der Anmeldung: **05.12.84 Patentblatt 84/49**

(45) Bekanntmachung des Hinweises auf die Patenterteilung: **04.10.89 Patentblatt 89/40**

(84) Benannte Vertragsstaaten: **AT BE CH DE FR GB IT LI LU NL SE**

(56) Entgegenhaltungen:
**I. CHIHATA, S. FUKUI, L.B. WIGGARD, ENZYME ENGINEERING, Band 6, 1982, Seiten 181-189, Plenum Press, New York, US J. KLEIN et al.: "New developments in the preparation and characterization of polymerbound biocatalysts"**
**DIE ANGEWANDTE MAKROMOLEKULARE CHEMIE, Band 104, 16. April 1982, Seiten 39-58, Hüthig & Wepf Verlag, Basel, CH; G. MANECKE et al.: "Immobilisierung von Corynebakterium simplex in thioliertem Polyvinylalkohol zur mikrobiologischen Steroidumwandlung"**
**DIE ANGEWANDTE MAKROMOLECULARE CHEMIE, Band 113, April 1983, Seiten 179-202, Hüthig & Wepf Verlag, Basel, CH; G. MANECKE et al.: "Immobilisierung von Corynebakterium simplex durch Photovernetzung von vinyliertem Polyvinylalkohol zur mikrobiologischen Steroidumwandlung**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Manecke, Georg, Prof. Dr., Hittorfstrasse 29, D-1000 Berlin 33 (DE)**
Erfinder: **Beier, Wilfried, Dipl.- Chem., Reuterplatz 1, D-1000 Berlin 44 (DE)**

EP 0 126 876 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft ein Verfahren zur Herstellung von 3-Oxo-$\Delta^{1,4}$-steroiden der allgemeinen Formel I:

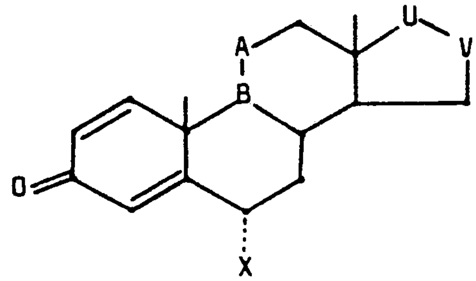

(I),

worin

X ein Wasserstoffatom, ein oder eine Methylgruppe bedeutet,

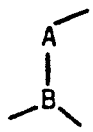

die Gruppierungen

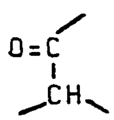

,

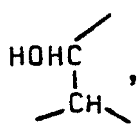

,

oder

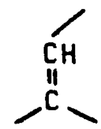

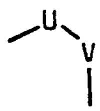

die Gruppierungen

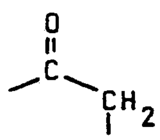

,

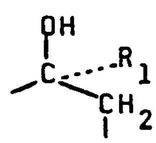

,

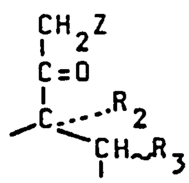

,

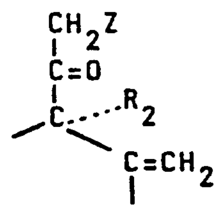

,

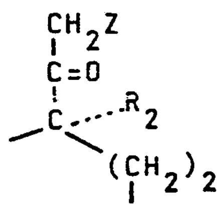

oder

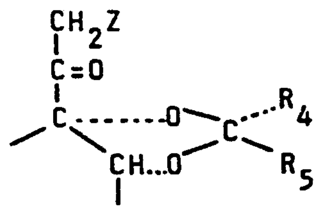

mit

Z in der Bedeutung eines Wasserstoffatoms, einer Hydroxygruppe eines Fluoratoms oder eines Chloratoms,

$R_1$ in der Bedeutung eines Wasserstoffatoms oder eines alicyclischen Kohlenwasserstoffrests mit 1 bis 4 Kohlenstoffatomen,

$R_2$ in der Bedeutung eines Wasserstoffatoms, einer Hydroxygruppe, einer Alkanoyloxygruppe mit 1 bis 6 Kohlenstoffatomen oder einer Benzoyloxygruppe,

$R_3$ in der Bedeutung eines Wasserstoffatoms, einer Hydroxygruppe oder einer Methylgruppe und

$R_4$ und $R_5$ in der Bedeutung einer Alkylgruppe mit 1 bis 4 Kohlenstoffatomen, darstellen, aus 3-Oxo-$\Delta^4$-steroiden der allgemeinen Formel II:

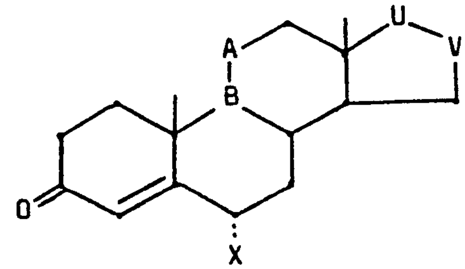

(II),

worin X, 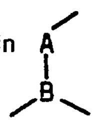 und 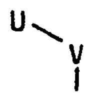 die obengenannte Bedeutung besitzen,

oder deren 21-Ester von Alkancarbonsäuren mit 1 bis 6 Kohlenstoffatomen durch Fermentation mit einem Biokatalysator in welchem Mikroorganismen der Spezies Arthrobacter simplex oder Bacillus sphaericus in vernetztem partiell mit Acrylsäure veresterten Polyvinylalkohol immobilisiert sind, welches dadurch gekennzeichnet ist, daß man den Biokatalysator einer wässrigen Suspension des mikronisierten 3-Oxo-$\Delta^4$-steroids zusetzt, die Reaktionsmischung bei einer Temperatur von 20 bis 40°C bis zur Umsetzung des Steroids inkubiert und danach den Biokatalysator mechanisch von der wässrigen Suspension des gebildeten 3-Oxo-$\Delta^{1,4}$-steroids abtrennt.

Bekanntlich werden Mikroorganismen-Kulturen der Species Arthrobacter simplex (wie z. B. ATCC 6946, 13260 und IFO 3530) oder Bacillus sphaericus (wie z. B. ATCC 7054, 7055, 12488 und 13805) zur $\Delta^1$-Dehydrierung von 3-Keto-$\Delta^4$-steroiden verwendet.

Es ist vorbekannt, daß Mikroorganismen der Species Arthrobacter simplex, die in vernetztem, partiell mit Acrylsäure veresterten Polyvinylalkohol immobilisiert sind, ebenfalls die Fähigkeit besitzen 3-Keto-$\Delta^4$-steroide zu 3-Keto-$\Delta^{1,4}$-steroiden zu dehydrieren (I. Chibata S. Fukui and L.B. Wingard: Enzyme Engineering Plenum Press, New York, Volume 6, 181-189). Auch aus Mikroorganismen der Species Bacillus sphaericus lassen sich in gleicher Weise Biokatalysatoren herstellen.

Diese für die Herstellung des Biokatalysators benötigten Mikroorganismen werden in üblicher Weise angezüchtet und nach erfolgter Anzucht abfiltriert oder abzentrifugiert, mit Wasser oder verdünnter Pufferlösung gewaschen und nochmals filtriert oder zentrifugiert. Die so erhaltene feuchte Biomasse kann ohne weitere Aufarbeitung zur Herstellung des Biokatalysators verwendet werden, wobei man etwa die 2 bis 30-fache vorzugsweise 10 bis 15-fache Menge feuchte Biomasse bezogen auf den vernetzten partiell mit Acrylsäure veresterten Polyvinylalkohol (Trockenmasse) verwendet. Andererseits kann aber diese Biomasse auch durch Sprühtrocknung getrocknet werden und man setzt dann 1 bis 10 mal vorzugsweise 5 bis 8 mal so viel Trockenpulver wie Polymerisat (Trockenmasse) ein.

Zur Herstellung des partiell mit Acrylsäure veresterten Polyvinylalkohols verwendet man vorzugsweise Polyvinylalkohol, welcher ein Molekulargewicht von 20 000 bis 200 000 hat.

Die Veresterung des Polyvinylalkohols mit Acrylsäure kann unter den Bedingungen durchgeführt werden, welche man üblicherweise zur Veresterung von primären oder sekundären Alkoholen mit sterisch ungehinderten Carbonsäuren anwendet.

Eine wenig aufwendige Veresterungsmethode ist ein Verfahren, bei dem der Polyvinylalkohol und Acrylsäure im molaren Verhältnis von etwa 1 : 1 bis 1 : 4 bezogen auf die Hydroxygruppen des Polyvinylalkohols in wässriger Lösung in Gegenwart starker Säuren - wie zum Beispiel Salzsäure, Schwefelsäure- oder stark saure Ionenaustauscher - wie zum Beispiel Amberlite®-IR-120 - erhitzt. Nach erfolgter Umsetzung wird die Säure - beispielsweise durch Neutralisation und anschließende Dialyse oder durch Kationenaustauscher, wie Amberlite®-IR-4B, entfernt - beziehungsweise den stark sauren Ionenaustauscher abfiltriert. Das erhaltene Produkt kann als Lösung oder nach Einengen im Vakuum oder Gefriertrocknen als Feststoff weiter umgesetzt werden. Der Feststoff pro g sollte etwa 0,4 bis 1,5 milliäquivalente Vinylgruppen enthalten. Die Bestimmung kann nach der in Methoden der organischen Chemie (Houben-Weyl-Müller Aufl. 4 Band II, Seite 53 beschriebenen Methode) erfolgen.

Die zum Zweck der Immobilisierung der Mikroorganismen folgende Vernetzung des mit Acrylsäure partiell veresterten Polyvinylalkohols erfolgt beispielsweise in Gegenwart eines Fotosensibilisators (wie z. B. Riboflavin-5'-monophosphat oder Natriumbenzoat) durch Bestrahlen mit sichtbarem oder UV-Licht.

Vorzugsweise wird der Mikroorganismus in einer eventuell gepufferten 1 bis 20 %-igen wässrigen Lösung des partiell mit Acrylsäure veresterten Polyvinylalkohols suspendiert, mit einem Fotosensibilisator versetzt, zu einer dünnen Schicht ausgebreitet und bestrahlt. Nach erfolgter Vernetzung wird der Biokatalysator gewaschen, gewünschtenfalls gefriergetrocknet und als Folie oder in zerkleinerter Form verwendet. Wird der Biokatalysator in zerkleinerter Form verwendet, so ist es zweckmäßig ihn nach der Zerkleinerung naß zu sieben und die Siebfraktionen von 0,02 bis 2 mm, vorzugsweise solche von 0,05 bis 1,5 mm Korngröße zur Umsetzung von Steroiden zu verwenden.

Zur Durchführung des erfindungsgemäßen Verfahrens werden pro 100 ml einer 0,1 bis 5 gewichtsprozentigen

Suspension des Biokatalysators in Wasser oder einer geeigneten Pufferlösung 0,03 bis 2 g des mikronisierten Steroids (die maximale Substratkonzentration ist abhängig von der Struktur des umzusetzenden Steroids) zugesetzt und man inkubiert die Reaktionsmischung bis zur Beendigung der Umsetzung. Oft ist es zweckmäßig das mikronisierte Steroid (Korngröße vorzugsweise 0,1 bis 50 µ zusätzlich noch zu emulgieren. Das kann geschehen, indem man es unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxyaddukte oder Fettsäureester von Polyglykolen. Als geeignete Emulgatoren seien die handelsüblichen Netzmittel Tegin®, Tagat®, Tween® und Span® beispielsmäßig genannt.

Die optimale Substratkonzentration, Substratzugabezeit und Fermentationsdauer ist von der Art der angewendeten Reaktionsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Nach erfolgter Fermentation wird der Biokatalysator mechanisch wie zum Beispiel mittels eines Siebes abgetrennt, gewaschen und kann dann erneut zur Fermentation eingesetzt werden.

Die nachfolgenden Ausführungsbeispiele dienen zur näheren Erläuterung des erfindungsgemäßen Verfahrens.

**Beispiel 1**

a) Zwei 2 l Erlenmeyerkolben von denen jeder 500 ml einer sterilisierten Nährlösung aus 0,5 % Corn steep, 0,1 % Hefeextrakt und 0,05 % Stärkezucker eingestellt auf pH 7,0 enthält, wird mit einer Lyophilkultur von Arthrobacter simplex (ATCC 6946) beimpft und 48 Stunden bei 30°C auf einem Rotationsschüttler geschüttelt. Diese Vorkultur dient zur Beimpfung eines 50 l Glasfermenters, der mit 30 l eines sterilisierten Mediums aus 0,5 % Corn steep, 0,1 % Hefeextrakt und 0,05 % Stärkezucker, eingestellt auf pH 7,0, gefüllt ist. Nach der Beimpfung wird bei 29°C unter Belüftung (10 Liter/Min.), 0,7 atü Druck und Rühren (220 Umdrehungen pro Minute) 24 Stunden germiniert. Mit dieser Vorfermenter-Kultur wird nun ein Stahlfermenter beimpft, der 500 l eines bei 121° C und 1,1 atü sterilisierten Mediums aus 0,5 % Corn steep, 0,1 % Hefeextrakt und 0,05 % Stärkezucker, eingestellt auf pH 7,0, enthält und unter den Bedingungen des Vorfermenters ebenfalls 24 Stunden bis zur optimalen Zelldichte germiniert.

Anschließend wird die Kultur in einem Labor Separator LG 205 [Firma Westfalia Separator AG/Ölde (Westf.)] bei 10 000 Umdrehungen/Min. separiert. Die abgeschleuderte Zellmasse wird zur Entfernung anhaftender Bestandteile des Nährmediums mit destillierten Wasser gewaschen und erneut zentrifugiert.

Die auf diese Weise dargestellte feuchte Bakterienpaste (2,85 kg) wird in 2,85 l eines 0,05 molaren Tris-(hydroxymethyl)-methylamin-Puffers von pH 7,6 aufgeschlemmt, zur Zerteilung von Zellklumpen durch ein Metallgazefilter gedrückt und in einem Universal-Labor-Zerstäubungstrockner (Firma Zahn & Co., Hameln, Deutschland) bei einer Verdampfungstemperatur von 80°C versprüht, wobei 600 ml Zellsuspension pro Stunde eingespeist werden.

Man erhält so 513 g Arthrobacter simplex Trockenpulver.

b) 22 g Polyvinylalkohol (Molgewicht ca. 72 000 Herstellerfirma Merck AG) wird in 250 ml in wässriger Salzsäure bei 80°C gelöst. Dann setzt man unter Stickstoff 72 g redestillierte Acrylsäure zu, erhitzt noch 3 Stunden lang bei 90°C unter Rühren und rührt weitere 24 Stunden lang bei Raumtemperatur.

Dann neutralisiert man die Reaktionsmischung mit verdünnter Natronlauge dialysiert sie zwei Tage lang gegen Wasser und lyophilisiert sie. (Der Vinylgruppengehalt des Polymeren liegt bei 0,80 m Äquiv./g.)

c) 10 ml einer 8 Gewichtprozentigen Lösung des gemäß b) hergestellten Polymeren in Phosphatpuffer (25 m mol Dinatriumhydrogenphosphat und 25 m mol Kaliumdihydrogenphosphat pro Liter) werden mit einer Suspension von 6,00 g Arthrobacter simplex Trockenpulver in 25 ml des gleichen Phosphatpuffers und 80 mg Riboflavin-5'-monophosphat-Natriumsalz versetzt, verrührt und auf einer 600 $cm^2$ großen Glasplatte gleichmäßig ausgestrichen. Man bestrahlt die Mischung 3 Stunden lang aus 20 cm Abstand mit einer 250 Watt starken Lampe mit sichtbarem Licht wobei die Temperatur im Strahlengang mittels eines Gebläses auf maximal 25°C begrenzt wird.

Der als Folie anfallende Biokatalysator wird 24 Stunden lang in Phosphatpuffer bei 30°C geschüttelt und dann lyophylisiert. Dann wird er zermahlen und die Teilchen mit einer Korngröße von 0,5 bis 1 mm ausgesiebt. Der so erhaltene Biokatalysator kann monatelang gelagert werden, ohne daß er in seiner Aktivität nachläßt.

d) Hydrocortison wird in einer Turbulenzmühle mikronisiert bis seine Teilchengröße überwiegend 1 bis 20 µ beträgt.

e) 36,2 mg mikronisiertes Hydrocortison werden in 50 ml Phosphat-Puffer (25 m mol Dinatriumhydrogenphosphat und 25 m mol Kaliumdihydrogenphosphat pro Liter) mit 100 mg lyophilisiertem Biokatalysator versetzt und bei 30°C mit 160 Umdrehungen pro Minute geschüttelt, bis zur vollständigen Umsetzung des Substrats (Dauer ca. 24 Stunden).

Nach beendeter Umsetzung wird der Biokatalysator von der Prednisolon-Suspension durch Sieben abgetrennt. In der gleichen Weise kann der Biokatalysator abermals mit mikronisierter Hydrocortison-Suspension umgesetzt werden. Selbst nach dreißigmaliger Wiederholung hat der Biokatalysator noch seine volle Aktivität beibehalten.

**Beispiel 2**

Unter den Bedingungen des Beispiels 1 können folgende Steroide in der $\Delta^1$-Position dehydriert werden:
4-Östren-3,17-dion (hier entsteht durch Doppelbindungsisomerisierung Östron),
4-Androsten-3,17-dion,
17β-Hydroxy-4-androsten-3-on,
4-Pregnen-3,20-dion,
17α-Hexanoyloxy-4-pregnen-3,20-dion,
11β,21-Dihydroxy-4-pregnen-3,20-dion,
17α,21-Dihydroxy-4-pregnen-3,11,20-trion,
11β,21-Dihydroxy-6α-methyl-17α-propionyloxy-4-pregnen-3,20-dion,
6α-Fluor-11β,21-dihydroxy-16α-methyl-4-pregnen-3,20-dion,
21-Hydroxy-16α,17α-isopropylidendioxy-4,11-pregnadien-3,20-dion
21-Hydroxy-17α-propionyloxy-16β-methyl-4,11-pregnadien-3,20-dion und

**Beispiel 3**

a) 22 g Polyvinylalkohol (Molgewicht ca. 72 000 Herstellerfirma Merck AG) wird in 250 ml in wässriger Salzsäure bei 80°C gelöst. Dann setzt man unter Stickstoff 36 g redestillierte Acrylsäure zu, erhitzt noch 3 Stunden lang bei 90°C unter Rühren und rührt weitere 24 Stunden lang bei Raumtemperatur.

Dann neutralisiert man die Reaktionsmischung mit verdünnter Natronlauge dialysiert sie zwei Tage lang gegen Wasser und lyophilisiert sie. (Der Vinylgruppengehalt des Polymeren liegt bei 0,40 m Äquiv./g.)

b) 10 ml einer 8 Gewichtprozentigen Lösung des gemäß b) hergestellten Polymeren in Phosphatpuffer (25 m mol Dinatriumhydrogenphosphat und 25 m mol Kaliumdihydrogenphosphat pro Liter) werden mit einer Suspension von 6,00 g Arthrobacter simplex - hergestellt nach Beispiel 1a) - Trockenpulver in 25 ml des gleichen Phosphatpuffers und 80 mg Riboflavin-5'-monophosphat-Natriumsalz versetzt, verrührt und auf einer 600 $cm^2$ großen Glasplatte gleichmäßig ausgestrichen. Man bestrahlt die Mischung 1 Stunde lang aus 40 cm Abstand mit einer 300 Watt starken UV-Fotolampe wobei die Temperatur im Strahlengang mittels eines Gebläses auf maximal 25°C begrenzt wird.

Der als Folie anfallende Biokatalysator wird 24 Stunden lang in Phosphatpuffer bei 30°C gewaschen.

c) Hydrocortison wird in einer Turbulenzmühle mikronisiert bis seine Teilchengröße überwiegend 1 bis 20 μ beträgt.

d) 36,2 mg mikronisiertes Hydrocortison werden in 50 ml Phosphat-Puffer (25 m mol Dinatriumhydrogenphosphat und 25 m mol Kaliumdihydrogenphosphat pro Liter) mit 400 mg der feuchten Biokatalysatorfolie versetzt und bei 30°C mit 160 Umdrehungen pro Minute geschüttelt, bis das Substrat völlig umgesetzt ist (Dauer ca. 30 Stunden).

Nach beendeter Umsetzung wird der Biokatalysator aus der Prednisolon-Suspension herausgenommen. In der gleichen Weise kann der Biokatalysator abermals mit mikronisierter Hydrocortison-Suspension umgesetzt werden. Selbst nach dreißigmaliger Wiederholung hat der Biokatalysator noch seine volle Aktivität beibehalten.

**Beispiel 4**

362 mg mikronisiertes Hydrocortison werden in 200 ml Phosphat-Puffer (25 m mol Dinatriumhydrogenphosphat und 25 m mol Kaliumdihydrogenphosphat pro Liter) suspendiert, mit 0,5 g lyophilisiertem Biokatalysator - hergestellt nach Beispiel 1 - versetzt und bei 30°C mit 160 Umdrehungen pro Minute bis zur völligen Umsetzung (ca. 48 Stunden lang) geschüttelt.

Dann wird der Biokatalysator durch Sieben abgetrennt, mit Phosphatpuffer vom Sieb gespült, mit einer Suspension von 362 mg mikronisiertem Hydrocortison versetzt, das Gemisch mit Phosphatpuffer auf 200 ml aufgefüllt und wiederum bis zur völligen Umsetzung des Substrats bei 30°C mit 160 Umdrehungen pro Minute geschüttelt.

Man wiederholt diese Umsetzung noch 9 mal mit dem gleichen Biokatalysator - wobei der Biokatalysator nach den einzelnen Umsetzungen wochenlang in Phosphatpuffer bei +4°C aufbewahrt werden kann, ehe er zur nächsten Umsetzung verwendet wird.

Die erhaltenen Prednisolon-Suspensionen werden jeweils mit Chloroform extrahiert und die organischen Phasen im Vakuum eingeengt.

Die vereinigten Prednisolon-Rohprodukte werden aus Aceton umkristallisiert. Man erhält 3,42 g Prednisolon (= 95 der Theorie) vom Schmelzpunkt 237 - 240°C.

**Patentanspruch**

Verfahren zur Herstellung von 3-Oxo-$\Delta^{1,4}$-steroiden der allgemeinen Formel I:

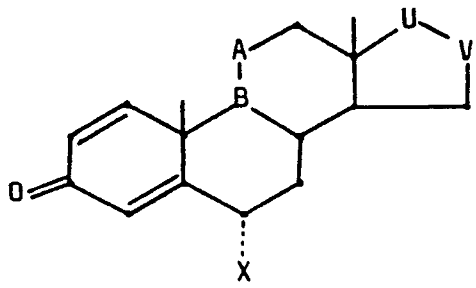

(I),

worin

X ein Wasserstoffatom, ein oder eine Methylgruppe bedeutet,

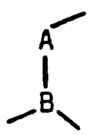

die Gruppierungen

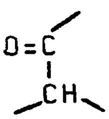

,

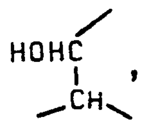

, oder

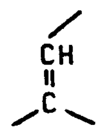

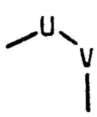

die Gruppierungen

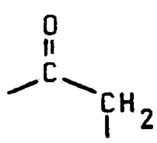

,

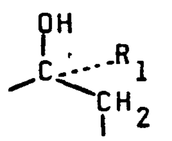

,

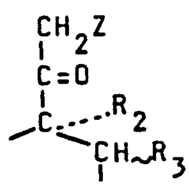

,

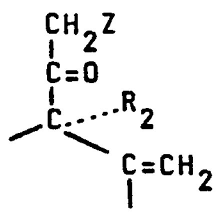

,

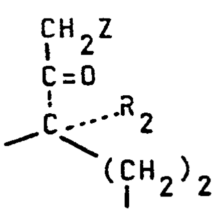

oder

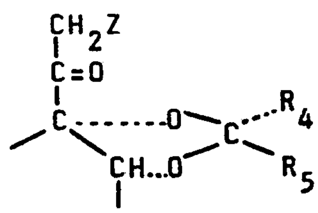

mit

Z in der Bedeutung eines Wasserstoffatoms, einer Hydroxygruppe eines Fluoratoms oder eines Chloratoms,

$R_1$ in der Bedeutung eines Wasserstoffatoms oder eines alicyclischen Kohlenwasserstoffrests mit 1 bis 4 Kohlenstoffatomen,

$R_2$ in der Bedeutung eines Wasserstoffatoms, einer Hydroxygruppe, einer Alkanoyloxygruppe mit 1 bis 6 Kohlenstoffatomen oder einer Benzoyloxygruppe,

$R_3$ in der Bedeutung eines Wasserstoffatoms, einer Hydroxygruppe oder einer Methylgruppe und

$R_4$ und $R_5$ in der Bedeutung einer Alkylgruppe mit 1 bis 4 Kohlenstoffatomen, darstellen, aus 3-Oxo-$\Delta^4$-steroiden der allgemeinen Formel II:

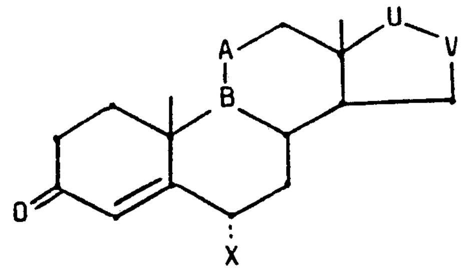

(II),

worin X, 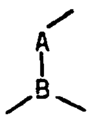 und 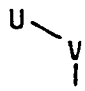 die obengenannte Bedeutung besitzen,

oder deren 21-Ester von Alkancarbonsäuren mit 1 bis 6 Kohlenstoffatomen durch Fermentation mit einem Biokatalysator in welchem Mikroorganismen der Spezies Arthrobacter simplex oder Bacillus sphaericus in vernetztem partiell mit Acrylsäure veresterten Polyvinylalkohol immobilisiert sind, dadurch gekennzeichnet, daß man den Biokatalysator einer wässrigen Suspension des mikronisierten 3-Oxo-$\Delta^{4}$-steroids zusetzt, die Reaktionsmischung bei einer Temperatur von 20 bis 40° C bis zur Umsetzung des Steroids inkubiert und danach den Biokatalysator mechanisch von der wässrigen Suspension des gebildeten 3-Oxo-$\Delta^{1,4}$-steroids abtrennt.

**Claim**

A process for the preparation of 3-oxo-$\Delta^{1,4}$-steroids of the general formula I:

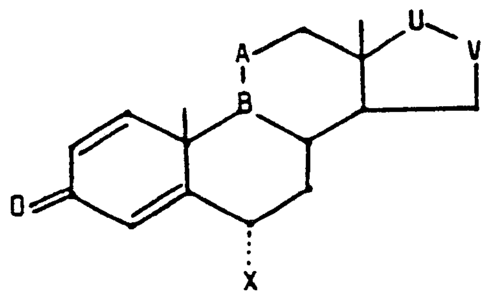

(I),

wherein
X is a hydrogen atom, a
or a methyl group,

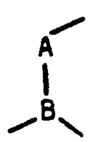 represents the groupings

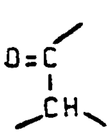 , 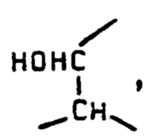 , or 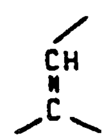

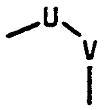 represents the groupings

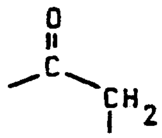 , 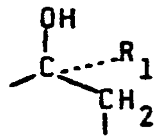 , 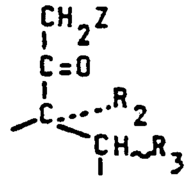 , 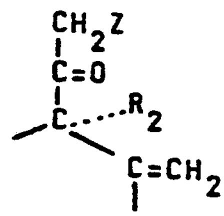 ,

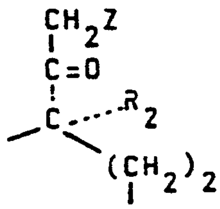 or 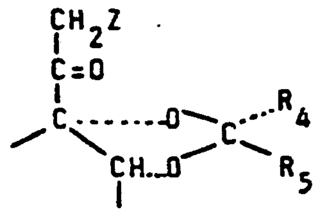

wherein

Z represents a hydrogen atom, a hydroxy group, a fluorine atom or a chlorine atom,

$R_1$ represents a hydrogen atom or an alicyclic hydrocarbon radical having from 1 to 4 carbon atoms,

$R_2$ represents a hydrogen atom, a hydroxy group, an alkanoyloxy group having from 1 to 6 carbon atoms, or a benzoyloxy group,

$R_3$ represents a hydrogen atom, a hydroxy group or a methyl group, and

$R_4$ and $R_5$ each represents an alkyl group having from 1 to 4 carbon atoms,

from 3-oxo-$\Delta^4$-steroids of the general formula II:

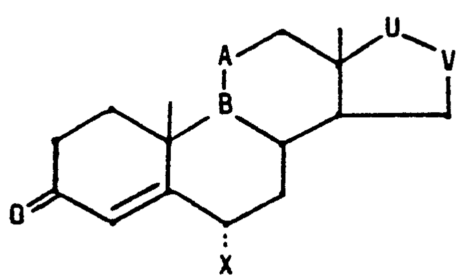 (II),

wherein X, 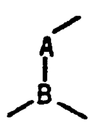 and 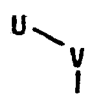 have the meanings given above,

or their 21-esters of alkanecarboxylic acids having from 1 to 6 carbon atoms, by fermentation with a biocatalyst in which microorganisms of the species Arthrobacter simplex or Bacillus sphaericus are immobilised in crosslinked polyvinyl alcohol partially esterified with acrylic acid, characterised in that the biocatalyst is added to an aqueous suspension of the micronised 3-oxo-$\Delta^4$-steroid, the reaction mixture is incubated at a temperature of from 20 to 40°C until the steroid has reacted, and then the biocatalyst is mechanically removed from the aqueous suspension of the 3-oxo-$\Delta^{1,4}$-steroid formed.

**Revendication**

Procédé pour préparer des oxo-3 $\Delta^{1,4}$-stéroïdes répondant à la formule générale I:

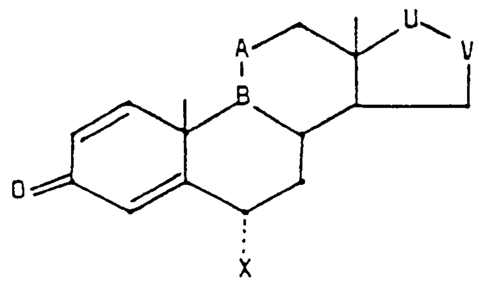

(I)

dans laquelle:

X représente un atome d'hydrogène ou un radical méthyle,

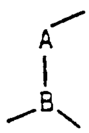 représente l'un des radicaux :

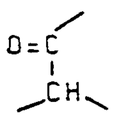 , 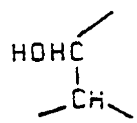 et 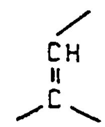

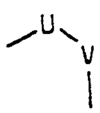 représente un radical répondant à l'une des formules suivantes :

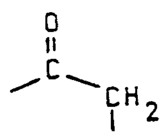 , 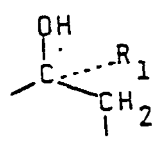 , 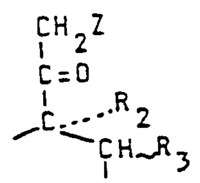 , 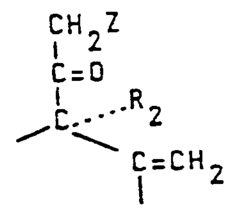 ,

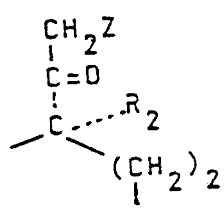 et 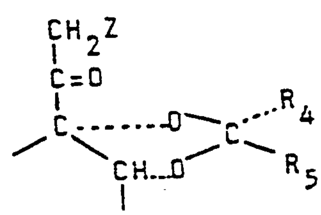

dans lesquelles:

Z représente un atome d'hydrogène un radical hydroxy, un atome de fluor ou un atome de chlore,

$R_1$ représente un atome d'hydrogène ou un radical hydrocarboné alicyclique contenant de 1 à 4 atomes de carbone,

$R_2$ représente un atome d'hydrogène, un radical hydroxy, un radical alcanoyloxy contenant de 1 à 6 atomes de carbone ou un radical benzoyloxy,

$R_3$ représente un atome d'hydrogène, un radical hydroxy ou un radical méthyle et

$R_4$ et $R_5$ représentent chacun un radical alkyle contenant de 1 à 4 atomes de carbone,

à partir d'oxo-3 $\Delta^4$-stéroïdes répondant à la formule générale II:

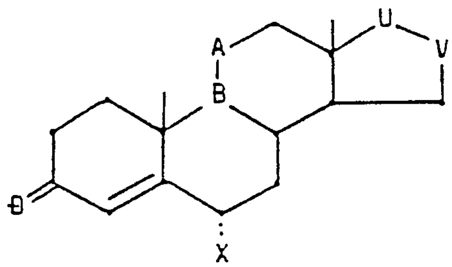

(II)

dans laquelle: X, 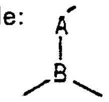 et 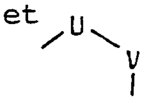

leur ont été données ci-dessus, ou à partir de leurs esters en 21 dérivant d'acides alcanoïques contenant de 1 à 6 atomes de carbone, par fermentation avec un biocatalyseur dans lequel des micro-organismes de l'espace Arthrobacter simplex ou Bacillus sphaericus sont immobilisés dans un poly-(alcool vinylique) réticulé, partiellement estérifié par l'acide acrylique, procédé caractérisé en ce qu'on ajoute le biocatalyseur à une suspension aqueuse de l'oxo-3 $\Delta^4$-stéroïde micronisé, on fait incuber le mélange réactionnel à une température comprise entre 20 et 40°C jusqu'à ce que le stéroïde ait réagi, puis on sépare le biocatalyseur mécaniquement de la suspension aqueuse de l'oxo-3 $\Delta^{1,4}$-stéroïde formé.